# EUROPÄISCHE PATENTSCHRIFT

(11) **EP 0 771 197 B1**
(45) Veröffentlichungstag und Bekanntmachung des Hinweises auf die Patenterteilung: **09.04.2003**
(21) Anmeldenummer: 95926896.2
(22) Anmeldetag: 14.07.1995
(51) Int. Cl.: A61K 31/33, A61K 31/445, A61K 31/495, A61P 25/18, A61P 25/24

(54) **VERWENDUNG HETEROCYCLISCHER VERBINDUNGEN ALS DOPAMIN-D 3 LIGANDEN**
USE OF HETEROCYCLIC COMPOUNDS AS DOPAMINES-D 3- LIGANDS
UTILISATION DE COMPOSES HETEROCYCLIQUES COMME LIGANDS DE LA DOPAMINE-D 3

(30) Priorität: 15.07.1994 DE 4425146
(43) Veröffentlichungstag der Anmeldung: 07.05.1997
(73) Patentinhaber: BASF AKTIENGESELLSCHAFT, 67056 Ludwigshafen (DE)
(72) Erfinder: HELLENDAHL, Beate, D-67105 Schifferstadt (DE); LANSKY, Annegret, D-64297 Darmstadt (DE); RENDENBACH-MÜLLER, Beatrice, D-67435 Neustadt (DE); BACH, Alfred, D-69123 Heidelberg (DE); UNGER, Liliane, D-67065 Ludwigshafen (DE); TESCHENDORF, Hans-Jürgen, D-67373 Dudenhofen (DE); WICKE, Carsten, D-67122 Altrip (DE)
(74) Vertreter: Kinzebach, Werner, Dr.
(86) Internationale Anmeldenummer: EP9502782
(87) Internationale Veröffentlichungsnummer: WO96002246

(56) Entgegenhaltungen:
- EP-A- 0 512 755
- EP-A- 0 558 245
- DE-A- 1 620 016
- DE-A- 2 060 816
- DE-A- 2 110 568
- DE-A- 2 213 808
- DE-A- 2 513 940
- US-A- 5 254 552
- US-A- 5 395 835
- BIOORG. MED. CHEM. LETT., Bd. 5, Nr. 3, Februar 1995 Seiten 219-222, MURRAY P. J. ET AL. 'A Novel Series of Arylpiperazines with High Affinity and Selectivity for the Dopamine D3 Receptor'

## Beschreibung

Die Erfindung betrifft die Verwendung heterocyclischer Verbindungen. Die erwähnten Verbindungen besitzen wertvolle therapeutische Eigenschaften und sind zur Behandlung von Schizophrenie, Depressionen, Neurosen und Psychosen brauchbar.

Verbindungen der hier in Rede stehenden Art mit physiologischer Aktivität sind bereits bekannt. So beschreibt die US-A-4,404,382 entsprechende Imidazolverbindungen mit anti-allergischer Aktivität.

Die US-A-3,362,956 beschreibt ebenfalls derartige Imidazolverbindungen. Diese besitzen adrenolytische und anti-konvulsive Aktivität.

Die DE-A-22 58 033 beschreibt Pyrazolverbindungen mit zentraldepressiver Aktivität.

Die DE-A-27 17 415 beschreibt Furan-, Thiophen-, Oxazol- und Thiadiazolverbindungen, die zur Behandlung von Überempfindlichkeitskrankheiten brauchbar sind.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der Etiologie von Schizophrenie und der Parkinson'schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

Sokoloff et al., Nature 1990, 347:146-151, hat einen dritten Subtyp gefunden, nämlich die D₃-Rezeptoren. Sie werden hauptsächlich im limbischen System exprimiert. Strukturell unterscheiden sich die D₃-Rezeptoren von den D₁- und D₂-Rezeptoren in etwa der Hälfte der Aminosäurereste.

Die Wirkung von Neuroleptika wurde im allgemeinen ihrer Affinität zu den D₂-Rezeptoren zugeschrieben. Neuere Rezeptorbindungs-studien haben dies bestätigt. Danach besitzen die meisten Dopaminantagonisten, wie Neuroleptika, hohe Affinität zu den D₂-Rezeptoren, aber nur geringe Affinität zu den D₃-Rezeptoren.

Bei den oben beschriebenen Verbindungen des Standes der Technik handelt es sich um solche D₂-Rezeptoragonisten bzw. -antagonisten.

Überraschenderweise wurde nun gefunden, daß die erfindungsgemäßverwendeten Verbindungen eine hohe Affinität zum Dopamin-D₃-Rezeptor und eine nur geringe Affinität zum D₂-Rezeptor aufweisen. Es handelt sich somit um selektive D₃-Liganden.

Gegenstand der vorliegenden Erfindung ist daher die Verwendung von Verbindungen der Formel I:

Het-A-B-Ar

worin
- A: für eine geradkettige oder verzweigte C₁-C₁₈-Alkylengruppe steht, die gegebenenfalls wenigstens eine Gruppe umfassen kann, die ausgewählt ist unter O, S, NR⁴, CONR⁴, NR⁴CO, COO, OCO und einer Doppel- oder Dreifachbindung,
- B: für einen Rest der Formel: 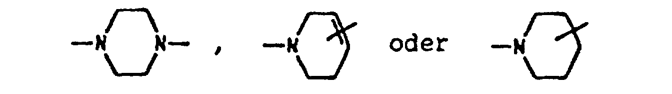 steht,
- Ar: für Phenyl, Pyridyl, Pyrimidyl oder Triazinyl steht, wobei Ar gegebenenfalls ein bis vier Substituenten aufweisen kann, die unabhängig voneinander ausgewählt sind unter OR⁴, C₁-C₈-Alkyl, C₂-C₈-Alkenyl, C₂-C₈-Alkinyl, Halogen, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter 0, S und N, wobei der carbocyclische oder der heterocyclische Ring gegebenenfalls substituiert ist durch C₁-C₈-Alkyl, Halogen, OC₁-C₈-Alkyl, OH, NO₂ oder CF₃, und wobei Ar gegebenenfalls auch mit einem carbocyclischen oder heterocyclischen Ring der oben definierten Art kondensiert sein kann,
- Het: für eine Gruppe steht, die ausgewählt ist unter 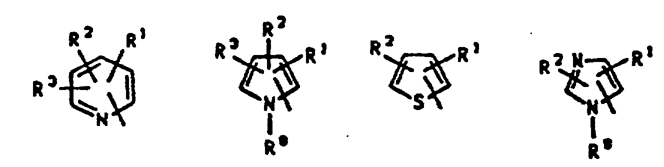 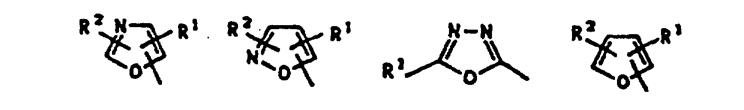 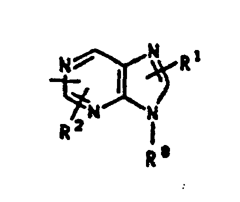
worin
R¹, R² und R³ unabhängig voneinander für H, Halogen, OR⁵, NR⁴R⁵, SR⁴, CF₃, CN, CO₂R⁴ oder C₁-C₈-Alkyl, das gegebenenfalls substituiert ist durch OH, OC₁-C₈-Alkyl oder Halogen stehen,
R⁴ für H oder C₁-C₈-Alkyl, das gegebenenfalls durch OH, OC₁-C₈-Alkyl oder Halogen substituiert ist, steht;
R⁵ die für R⁴ angegebenen Bedeutungen besitzt oder für COR⁴ oder CO₂R⁴ steht;
R⁸ die für R⁵ angegebenen Bedeutungen besitzt,
und der Salze davon mit physiologisch verträglichen Säuren,
zur Herstellung eines pharmazeutischen Mittels zur Behandlung von Schizophrenie, Depressionen, Neurosen und Psychosen.

Bei den erfindungsgemäßverwendeten Verbindungen handelt es sich um selektive Dopamin-D₃-Rezeptor-Liganden, die regioselektiv im limbischen System angreifen. Sie sind aufgrund ihrer geringen Affinität zum D₂-Rezeptor nebenwirkungsärmer als die klassichen Neuroleptika, bei denen es sich um D₂-Antagonisten handelt. Die Verbindungen sind daher zur Behandlung von Erkrankungen brauchbar, die auf Dopamin-D₃-Rezeptorantagonisten bzw. -agonisten ansprechen, wie hier zur Behandlung von Erkankungen des zentralen Nervensystems, mämlich Schizophrenie, Depressionen, Neurosen und Psychosen. Außerdem sind sie zur Behandlung von Schlafstörungen und Übelkeit und als Antihistaminika brauchbar.

Im Rahmen der vorliegenden Erfindung besitzen die nachfolgenden Ausdrücke die anschließend angegebenen Bedeutungen:

Alkyl (auch in Resten wie Alkoxy, Alkylamino etc.) bedeutet eine geradkettige oder verzeigte Alkylgruppe mit 1 bis 8 Kohlenstoffatomen, vorzugsweise 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH und OC₁-C₈-Alkyl.

Beispiele für eine Alkylgruppe sind Methyl, Ethyl, n-Propyl, i-Propyl, n-Butyl, iso-Butyl, t-Butyl etc.

Alkylen steht für geradkettige oder verzweigte Reste mit vorzugsweise 2 bis 10 Kohlenstoffatomen, besonders bevorzugt 3 bis 8 Kohlenstoffatomen und insbesondere 3 bis 6 Kohlenstoffatomen.

Die Alkylengruppen können gegebenenfalls wenigstens eine der oben angegebenen Gruppen umfassen. Diese kann - ebenso wie die erwähnte Doppel- oder Dreifachbindung - in der Alkylenkette an beliebiger Stelle oder am Ende der Kette angeordnet sein, so daß sie die Kette mit dem heterocyclischen Rest verbindet. Letzteres ist bevorzugt. Wenn die Alkylengruppe eine Doppel- oder Dreifachbindung umfaßt, besitzt sie mindestens drei Kohlenstoffatome in der Kette.

Halogen bedeutet F, Cl, Br, I und insbesondere Cl, Br, I.

Der Rest Ar kann einen, zwei, drei oder vier Substituenten aufweisen. Die Substituenten können an jeder Position des Phenylrings angeordnet sein. Vorzugsweise befindet sich jedoch mindestens einer in m-Position.

Vorzugsweise sind sie unabhängig voneinander ausgewählt unter H, C₁-C₈-Alkyl, OC₁-C₈-Alkyl, CHF₂, CF₃, CN, Halogen, SO₂OR⁴ und CO₂R⁴.

Ar weist vorzugsweise wenigstens einen Substituenten auf und steht insbesondere für 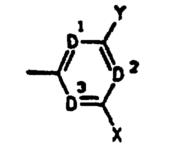 worin D¹, D² und D³ unabhängig voneinander für CH oder N stehen und X und Y für H oder die oben bzw. nachfolgend angegebenen Bedeutungen stehen.

Vorzugsweise stehen D¹, D² und D³ für CH oder D¹ für N und D² und D³ für CH. Wenn einer der Substituenten des Restes Ar für einen 5- oder 6-gliedrigen heterocyclischen Ring steht, so handelt es sich beispielsweise um eine Pyrrolidin-, Piperidin-, Morpholin-, Piperazin-, Pyridin-, Pyrimidin-, Triazin-, Pyrrol-, Thiophen-, Thiazol-, Imidazol-, Oxazol-, Isoxazol-, Pyrazol-, oder Thiadiazolrest.

Wenn einer der Substituenten des Restes Ar für einen carbocyclischen Rest steht, handelt es sich insbesondere um einen Phenyl-, Cyclophenyl- oder Cyclohexylrest.

Wenn einer der Substituenten des Restes Ar für C₁-C₈-Alkyl steht, ist ein verzweigter Rest, insbesondere die Isopropyl- oder t-Butylgruppe, bevorzugt.

Wenn Ar mit einem carbocyclischen oder heterocyclischen Rest kondensiert ist, steht Ar insbesondere für einen Naphthalin-, Dioder Tetrahydronaphthalin-, Chinolin-, Di- oder Tetrahydro-chinolin, Indol-, Dihydroindol-, Benzimidazol-, Benzothiazol-, Benzothiadiazol-, Benzopyrrol- oder Benzotriazolrest.

Eine bevorzugte Ausführungsform sind die Verbindungen der Formel I, worin A für C₁-C₈-Alkylen steht, das ein Sauerstoff- oder Schwefelstoffatom oder die Gruppe CONR⁴, insbesondere O oder S, umfassen kann.

Eine weitere bevorzugte Ausführungsform sind die Verbindungen der Formel I, worin Het für eine Gruppe der folgenden allgemeinen Formeln steht:

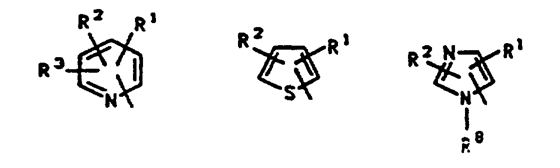

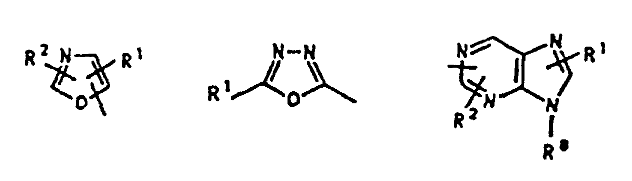

Eine weitere bevorzugte Ausführungsform sind die Verbindungen der Formel I, worin Het für eine Gruppe der folgenden allgemeinen Formeln steht:

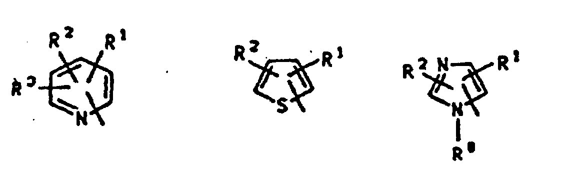

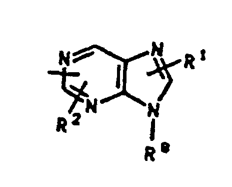

Eine weitere bevorzugte Ausführungsform sind die Verbindungen der Formel I, worin Het für eine Gruppe der allgemeinen Formeln steht:

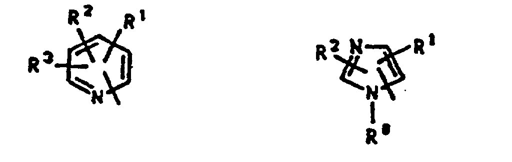

R¹, R², R³ und R⁸ besitzen dabei stets die oben angegebenen Bedeutungen.

vorzugsweise stehen R¹, R², und R³ unabhängig voneinander für H; NR⁴R⁵, OR⁵, C₁-C₈-Alkyl, CO₂R⁴, CF₃ oder Halogen.

Vorzugsweise weist der Rest Het einen oder zwei, insbesondere einen Substituenten auf.

Wenn Het für einen Pyridinrest steht, sind R¹, R², und R³ vorzugsweise und unabhängig ausgewählt unter H, Halogen, OR⁵, NR⁴R⁵, CF₃, CO₂R⁴ und C₁-C₈-Alkyl.

Wenn Het für einen Thiophenrest steht, sind R¹ und R² vorzugsweise und unabhängig ausgewählt unter Halogen und C₁-C₈-Alkyl.

Wenn Het für einen Purinrest steht, dann steht A vorzugsweise für S-C₄-C₇-Alkyl.

Gemäß einer weiteren Ausführungsform steht A für eine C₃-C₆-Alkylengruppe, die gegebenenfalls S, O oder CONR⁴ umfassen kann.

X steht vorzugsweise für H, CF_{3,} CN, Halogen, NO₂, CHF₂, C₁-C₈-Alkyl, insbesondere C₂-C₄-Alkyl, SO₂R⁴ oder CO₂R⁴ und insbesondere für H, CF₃, Halogen, CHF₂, C₁-C₈-Alkyl oder CN. Besonders bevorzugt steht X für CF₃, CHF₂ oder C₂-C₄-Alkyl.

Y steht vorzugsweise für C₁-C₈-Alkyl, insbesondere C₂-C₄-Alkyl, oder Wasserstoff.

Eine besonders bevorzugte Ausführungsform sind die Verbindungen der Formel Ia: 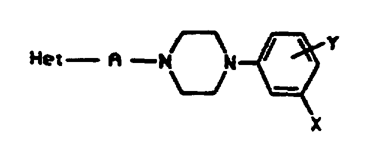 und insbesondere die Verbindungen der Formel Ib: 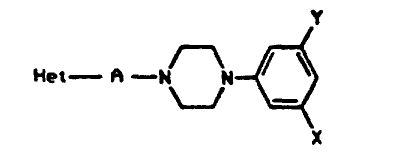 worin A, Het, X und Y die oben angegebenen Bedeutungen besitzen. Insbesondere stehen in den Formeln Ia und Ib X für CF₃ und Y für H bzw. X und Y beide für C₁-C₈-Alkyl.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Verbindungen der Formeln I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Soweit die Verbindungen der Formel I neu sind, erfolgt ihre Herstellung analog zu dem eingangs erwähnten Stand der Technik nach dem Fachmann geläufigen Methoden.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung, ohne sie zu begrenzen.

### Beispiel 1

### 2-[3-(4-{3-Trifluormethylphenyl}piperazinyl)-propylthio]-pyridin

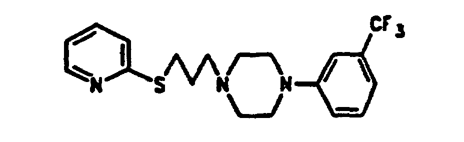

a) 1-(3-Chlorpropyl)-4-(3-trifluormethylphenyl)piperazin
   30 g (0,13 mol) Trifluormethylphenylpiperazin, 23 g (0,146 mol) 1,3-Bromchlorpropan und 15 g (0,148 mol) Triethylamin wurden in 200 ml THF 4 Stunden unter Rückfluß erhitzt. Nach Abkühlen wurde abgesaugt und eingeengt. Der zähflüssige Rückstand wurde mit Essigester aufgenommen, mit Wasser gewaschen, über MgSO₄ getrocknet und anschließend eingeengt. Als Rückstand erhielt man 39 g Produkt als gelbliches öl (quantitative Ausbeute).
b) 2-[3-(4-{Trifluormethylphenyl}piperazinyl)-propylthio]-pyridin
   1,11 g (10 mmol) 2-Mercaptopyridin, 3,1 g (10,1 mmol) 1-(3-Chlorpropyl)-4-(3-trifluormethylphenyl)piperazin und 1,5 g (15 mmol) Triethylamin wurden in 5 ml DMF 1 Stunde bei 100°C gerührt. Anschließend wurde auf 5%-ige Salzsäure gegossen und mit Essigester extrahiert. Nach Alkalisieren der wäßrigen Phase mit Natronlauge wurde wieder mit Essigester extrahiert, die organische Phase über MgSO₄ getrocknet und eingeengt. Der Rückstand wurde chromatographisch gereinigt (Laufmittel : CH₂Cl₂/CH₃OH = 98/2). Es wurden 2,5 g Produkt als gelbliches öl erhalten (= 65 % Ausbeute).
H-NMR [δ,ppm]:1,95 (2H); 2,55 (2H); 2,62 (4H); 3,23 (6H); 6,95 (1H); 7,05 (3H); 7,17 (1H); 7,36 (1H); 7,48 (1H); 8,42 (1H)

### Beispiel 2

### 2-[5-(4-{3-Trifluormethylphenyl}piperazinyl)-pentylmercapto]-pyridin

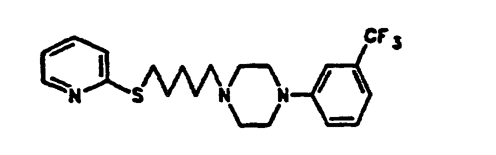

a) 2-(5-Chlorpentylmercapto)-pyridin
   2,78 g (25 mmol) 2-Mercaptopyridin, 4,64 g (25 mmol) 1,5-Bromchlorpentan und 2,58 g (25,5 mmol) Triethylamin wurden in 100 ml THF 4 Stunden unter Rückfluß erhitzt. Nach dem Abkühlen wurde abgesaugt, eingeengt und der Rückstand chromatographisch gereinigt (Laufmittel : Cyclohexan/Essigester = 92/8). Man erhielt 4 g Produkt (= 74 % Ausbeute).
b) 2-[5-(4-{3-Trifluormethylphenyl}piperazinyl)-pentylmercapto]-pyridin
   2,37 g (11 mmol) 2-(5-Chlorpentylmercapto)-pyridin, 2,78 g (12 mmol) m-Trifluormethylphenylpiperazin und 1,22 g (12,1 mmol) Triethylamin wurden in 5 ml DMF 5 Stunden bei 90°C gerührt. Anschließend wurde auf Wasser gegossen und dreimal mit CH₂Cl₂ extrahiert, über MgSO₄ getrocknet und eingeengt. Der Rückstand wurde mit Methyl-t-butylether versetzt, abgesaugt und die Mutterlauge eingeengt. Nach chromatographischer Reinigung (Laufmittel : CH₂Cl₂/CH₃OH = 96/4) erhielt man 3,0 g Produkt als Öl (= 67 % Ausbeute).
H = NMR [δ; ppm]: 1,5 (4H); 1,75 (2H); 2,4 (2H); 2,6 (4H); 3,2 (2H); 3,25 (4H); 7,0 (1H); 7,1 (3H); 7,2 (1H); 7,35 (1H); 7,45 (1H); 8,4 (1H)

### Beispiel 3

### 3-(4-{3-Trifluormethylphenyl}piperazinyl)-propylaminocarbonyl]thiophen

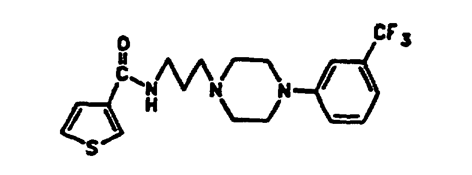

Eine Mischung aus 0,76 g (5,9 mmol) 3-Thiophencarbonsäure, 1,0 g (6,2 mmol) Carbonyldiimidazol und 1 Spatelspitze Dimethylaminopyridin in CH₂Cl₂ wurde 1/2 h bei Raumtemperatur gerührt. Zu dieser Mischung wurden 1,9 g (5,9 mmol) N-3-(Trifluormethylphenyl)-N-3-aminopropyl-piperazin getropft und über Nacht bei Raumtemperatur weitergerührt. Nach wäßriger Aufarbeitung wurde an SiO₂ (Laufmittel : CH₂Cl₂/CH₃OH = 10:1) chromatographiert. Das erhaltene Öl wurde in wenig CH₃OH gelöst. Durch Zugabe von 0,64 g (5,5 mmol) Fumarsäure in CH₂Cl₂ erhielt man 1,3 g Produkt als weißen Feststoff. Schmp.: 124 - 125°C.

### Beispiel 4

### 2-[2-{4-{3-Trifluormethylphenyl}piperazinyl)-ethylaminocarbonyl]-pyridin

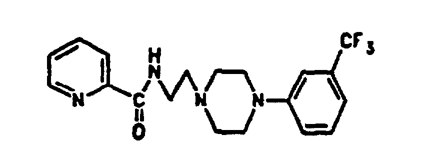

Zu einer Lösung von 0,95 g 2-Pyridincarbonsäure und 1,1 ml NEt₃ in CH₂Cl₂ wurden bei 0°C 0,74 ml Chlorethylformiat getropft. Nach 15 min Rühren bei Raumtemperatur wurde erneut gekühlt und 2 g N-3-Trifluormethylphenyl-N-2-aminoethyl-piperazin wurden zugetropft. Es wurde noch 3 h bei Raumtemperatur gerührt, mit H₂O, NH₄Cl-Lösung, NaOH, H₂O gewaschen, über MgSO₄ getrocknet und eingeengt. Nach Umkristallisation aus Essigester/Heptan erhielt man 1,9 g Produkt. Schmp.: 108 - 110°C.

### Beispiel 5

### 2-[3-(4-{3-Trifluormethylphenyl)phenyl)piperazinyl)propylamino-carbonylmethyl)-pyridin

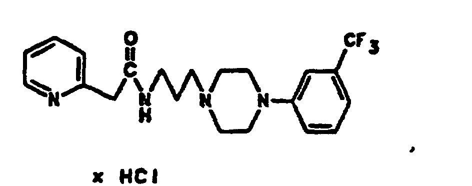

Zu einer Lösung von 2,34 g 2-Pyridinessigsäure-(N-hydroxysuccinimid)ester in CH₂Cl₂ wurden unter Kühlung 2,87 g N-3-Trifluormethylphenyl-N-3-aminopropyl-piperazin getropft. Die Mischung wurde über Nacht bei Raumtemperatur gerührt, anschließend mit NaHCO₃-Lösung und Wasser gewaschen. Die organische Phase wurde abgetrennt, mit MgSO₄ getrocknet und das Lösungsmittel abdestilliert. Zum Rückstand wurde wenig CH₃OH gegeben und anschließend wurde tropfenweise mit etherischer HCl versetzt. Man erhielt 2,0 g Produkt als weißen Feststoff. Schmp: 178 - 179°C.

In analoger weise wurden folgende Verbindungen synthetisiert:

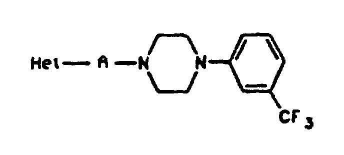

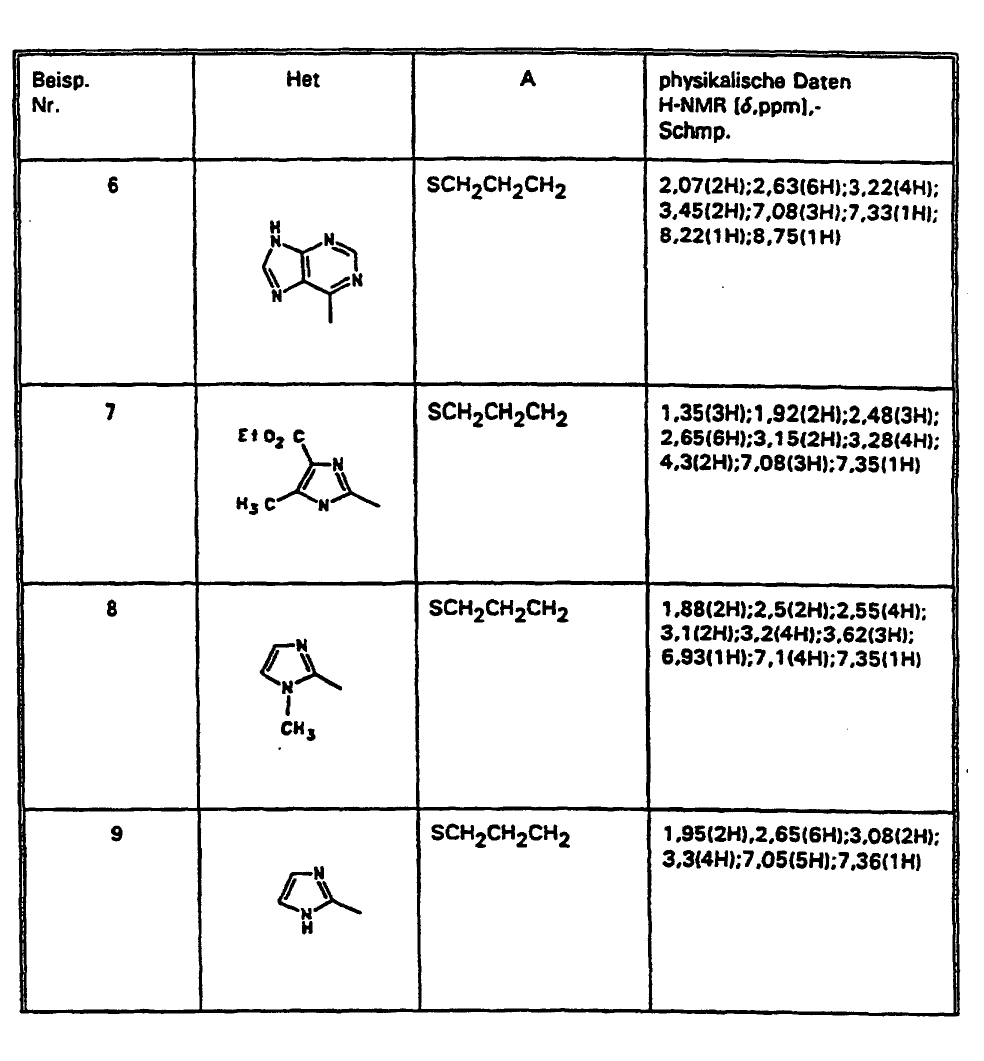

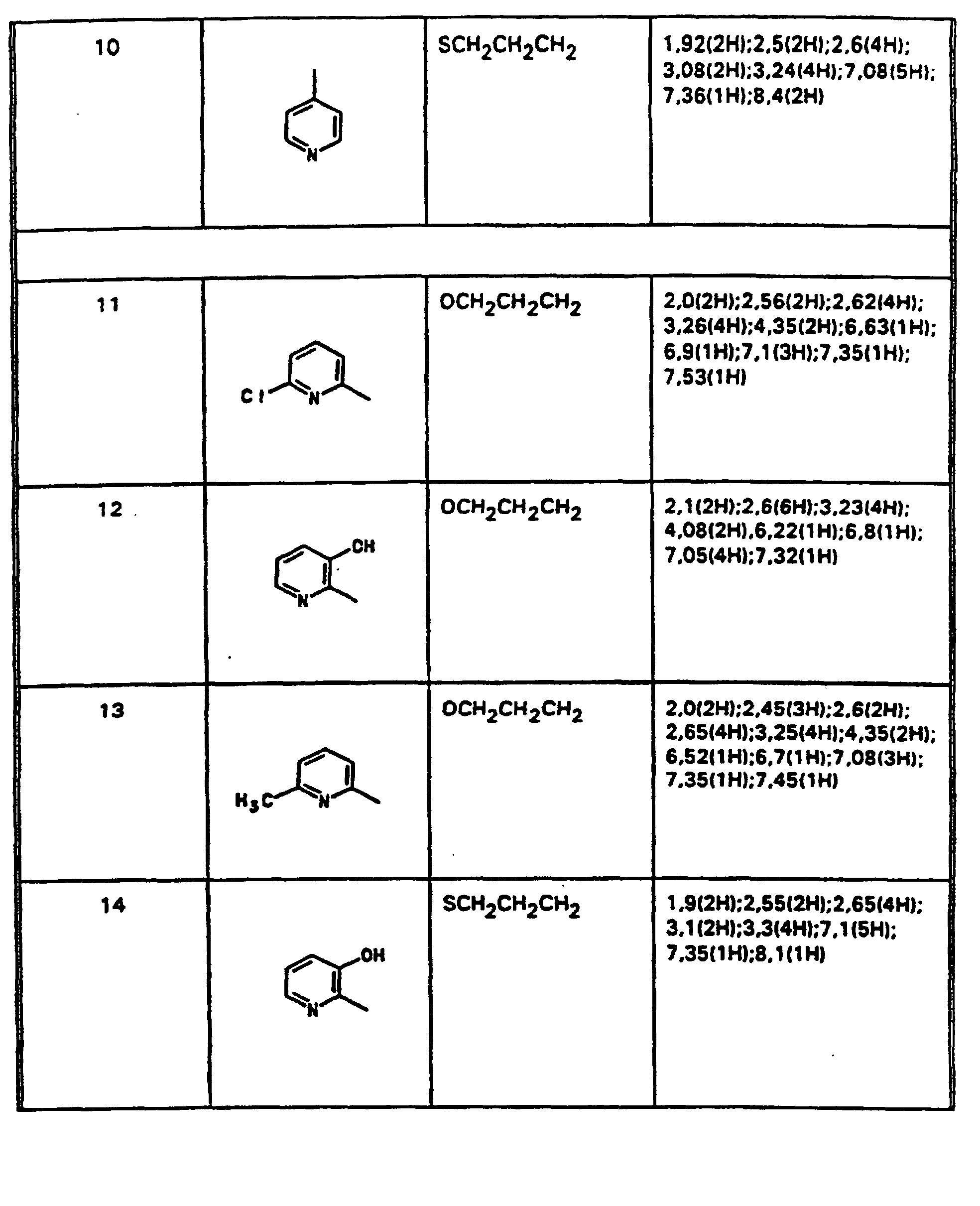

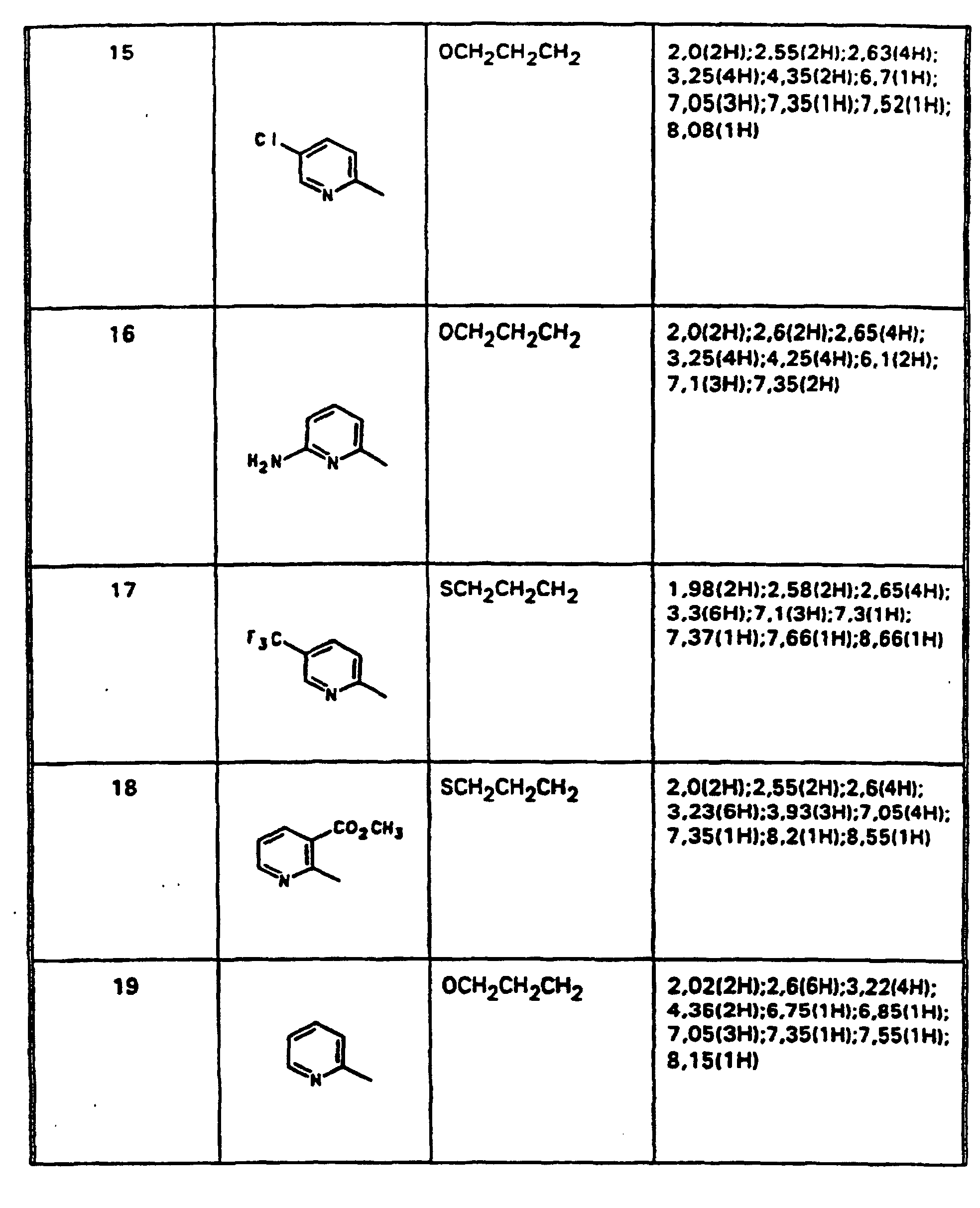

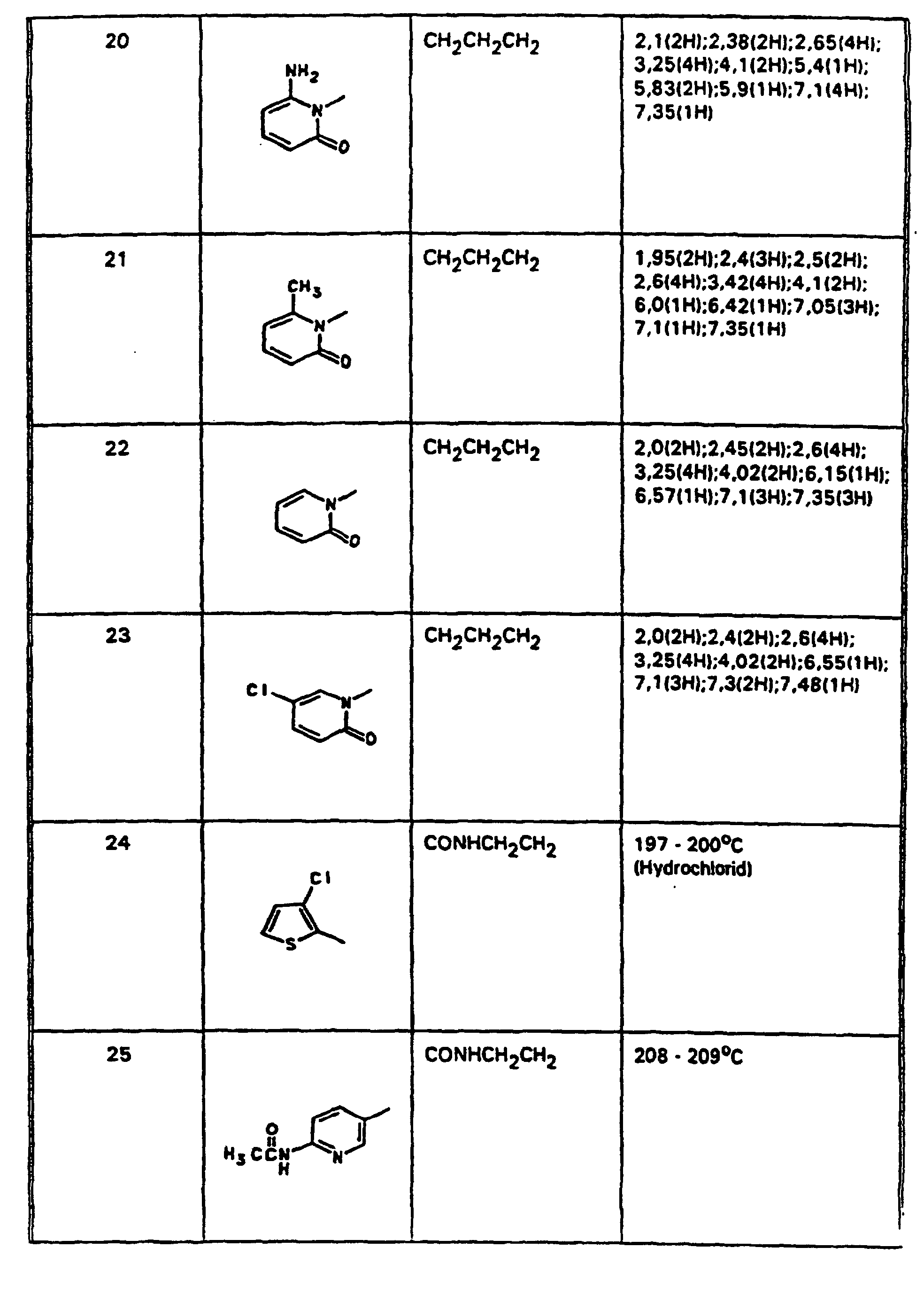

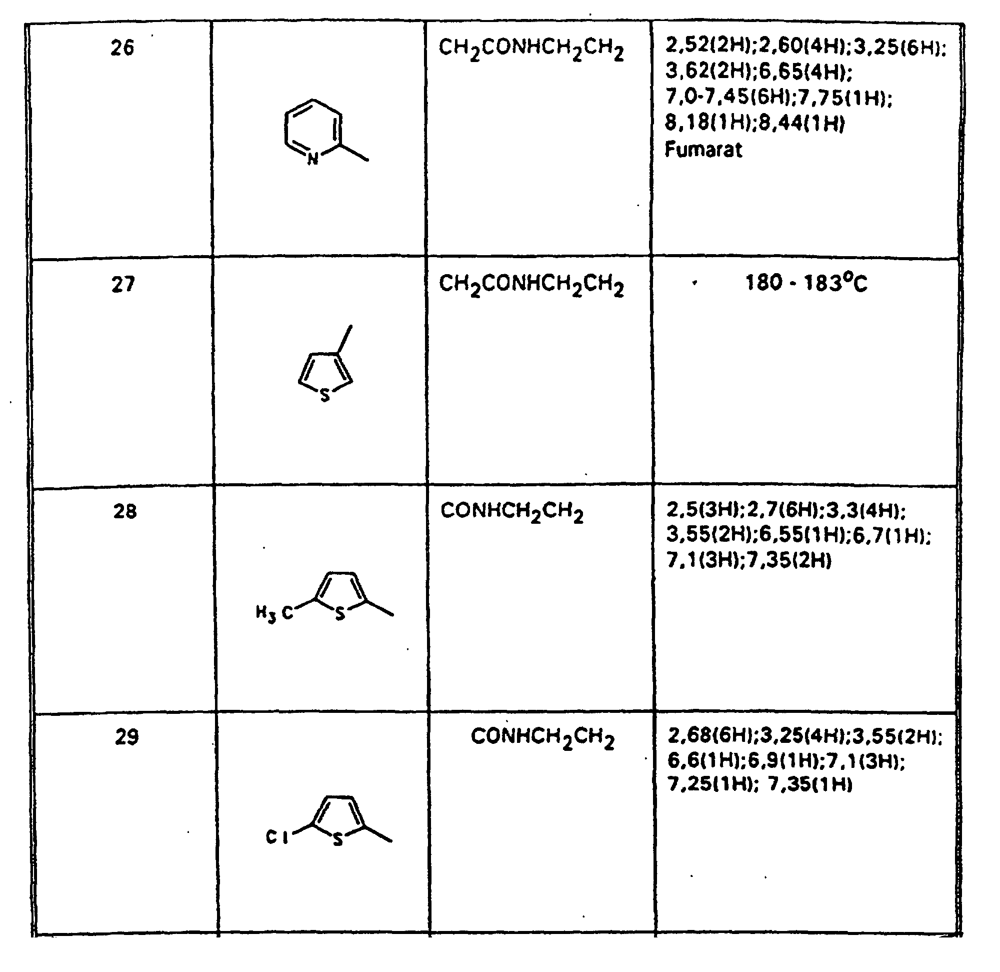

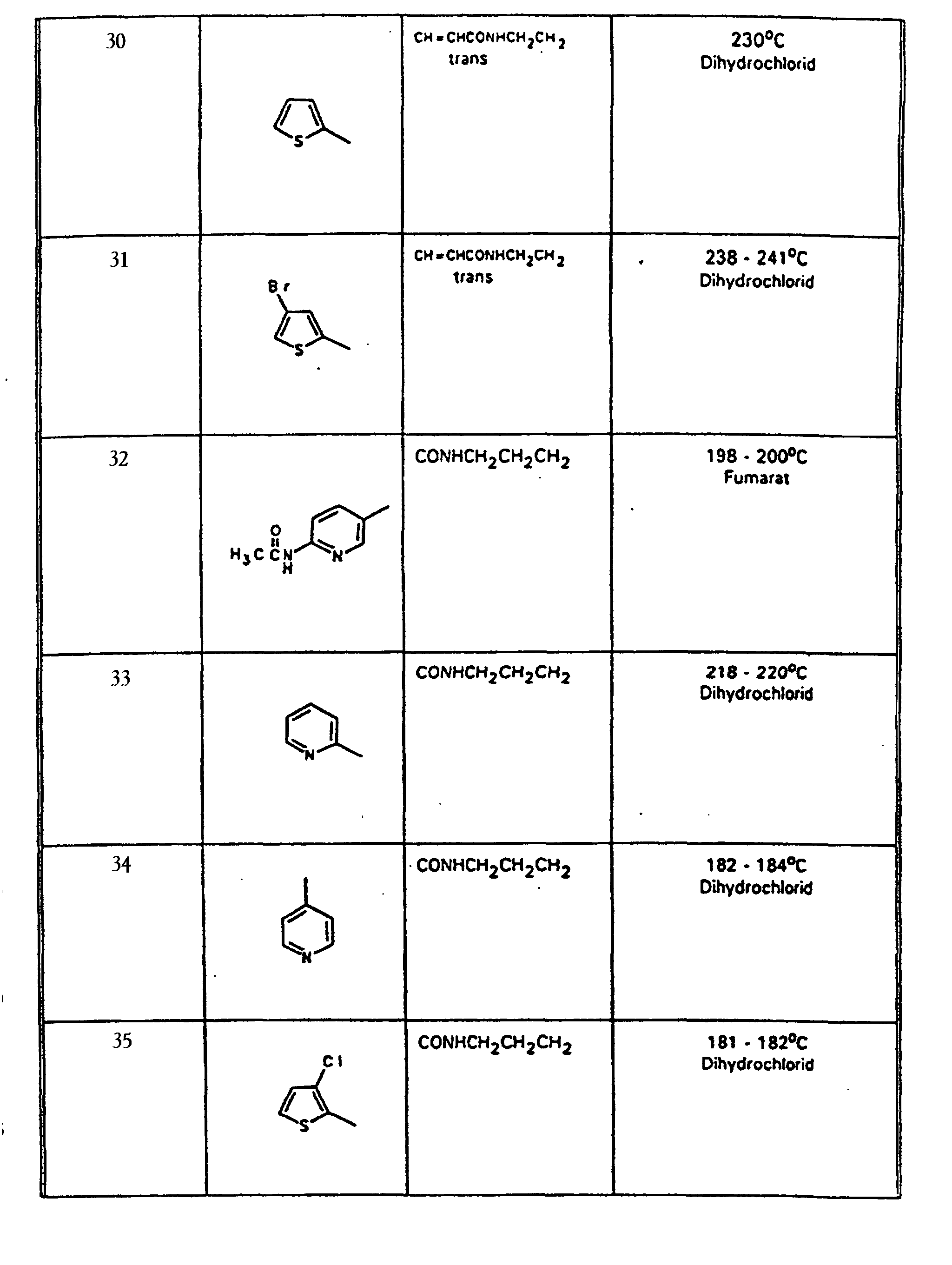

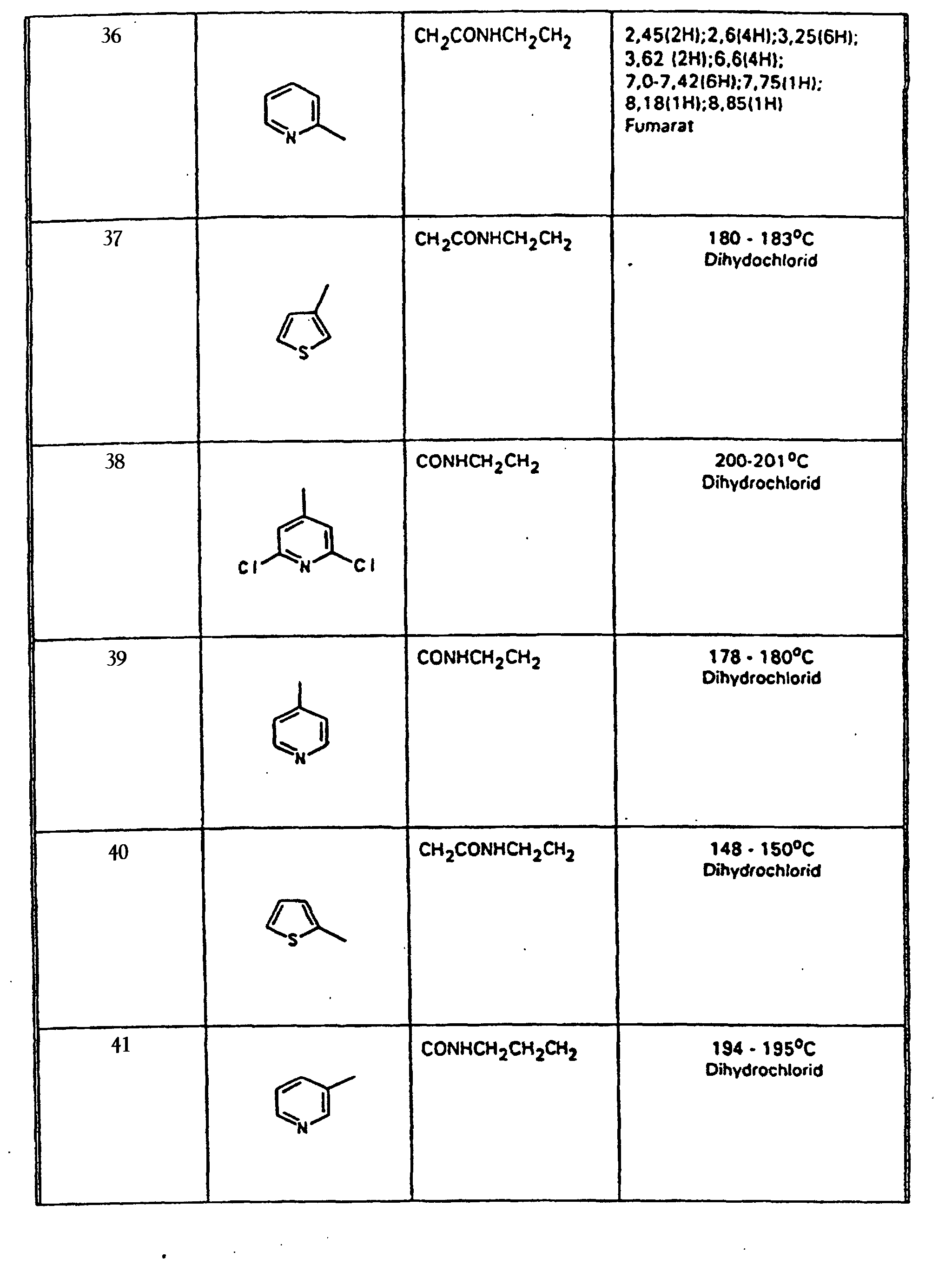

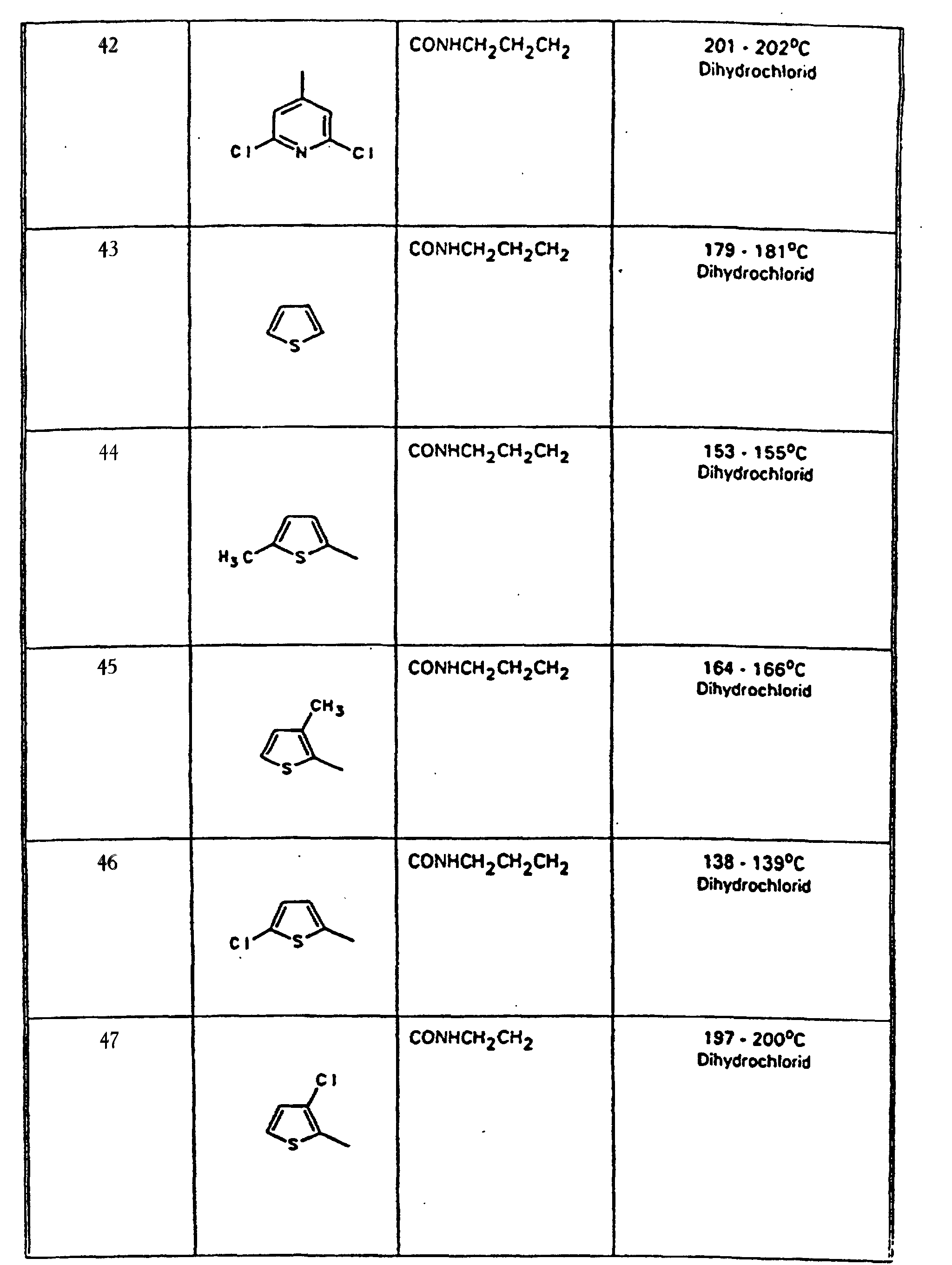

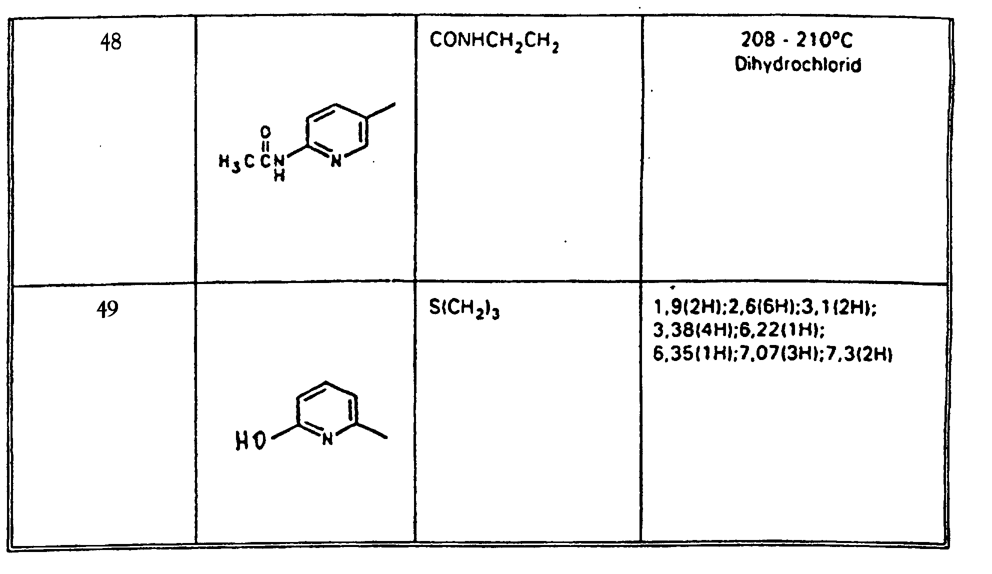

### Beispiele für galenische Applikationsformen:

### A) Tabletten

Auf einer Tablettenpresse werden in üblicher Weise Tabletten folgender Zusammensetzung gepreßt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### B) Dragees

| | |
|---|---|
| 20 mg | Substanz des Beispiels 4 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen

### Rezeptorbindungsstudien

Für Bindungsstudien wurden klonierte humane D₃-Rezeptor-exprimierende CCl 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 xg gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷ Zellen/ml Lysispuffer 30 min bei 4°C inkubiert. Die Zellen wurden bei 200 xg 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Pakkard 2200 CA Flüssigkeitszintillationszähler quantifiziert.

Die Bestimmung der K₁-Werte erfolgte über nichtlineare Regressionsanalyse mit dem Programm LIGAND. Die erfindungsgemäßen Verbindungen zeigen in diesem Test sehr gute Affinitäten zum D₃-Rezeptor bei guten Selektivitäten gegenüber dem D₂-Rezeptor.

## Patentansprüche

1. Verwendung der Verbindungen der Formel I:
Het-A-B-Ar
worin
A für eine geradkettige oder verzweigte C₁-C₁₈-Alkylengruppe steht, die gegebenenfalls wenigstens eine Gruppe umfassen kann, die ausgewählt ist unter O, S, NR⁴, CONR⁴, NR⁴CO, COO, OCO und einer Doppel- oder Dreifachbindung,
B für einen Rest der Formel: 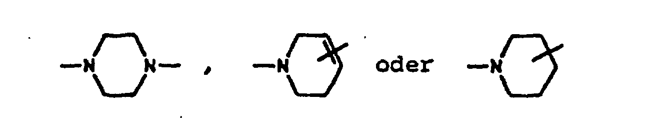 steht,
Ar für Phenyl, Pyridyl, Pyrimidyl oder Triazinyl steht, wobei Ar gegebenenfalls ein bis vier Substituenten aufweisen kann, die unabhängig voneinander ausgewählt sind unter OR⁴, C₁-C₈-Alkyl, C₂-C₈-Alkenyl, C₂-C₈-Alkinyl, Halogen, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter 0, S und N, wobei der carbocyclische oder der heterocyclische Ring gegebenenfalls substituiert ist durch C₁-C₈-Alkyl, Halogen, OC₁-C₈-Alkyl; OH, NO₂ oder CF₃, und wobei Ar gegebenenfalls auch mit einem carbocyclischen oder heterocyclischen Ring der oben definierten Art kondensiert sein kann,
Het für eine Gruppe steht, die ausgewählt ist unter 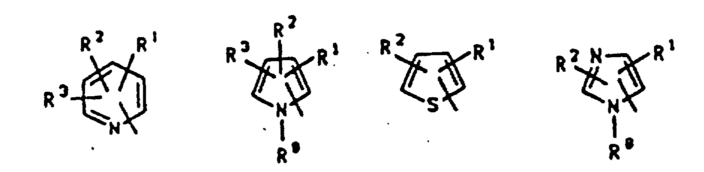 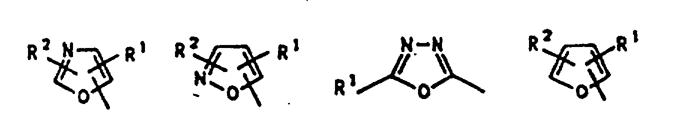 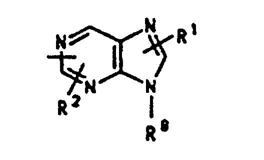
worin
R¹, R² und R³ unabhängig voneinander für H, Halogen, OR⁵, NR⁴R⁵, SR⁴, CF₃, CN, CO₂R⁴ oder C₁-C₈-Alkyl, das gegebenenfalls substituiert ist durch OH, OC₁-C₈-Alkyl oder Halogen stehen,
R⁴ für H oder C₁-C₈-Alkyl, das gegebenenfalls durch OH, OC₁-C₈-Alkyl oder Halogen substituiert ist, steht;
R⁵ die für R⁴ angegebenen Bedeutungen besitzt oder für COR⁴ oder CO₂R⁴ steht;
R⁸ die für R⁵ angegebenen Bedeutungen besitzt,
und der Salze davon mit physiologisch verträglichen Säuren,
zur Herstellung eines pharmazeutischen Mittels zur Behandlung von Schizophrenie, Depressionen, Neurosen und Psychosen.

2. Verwendung nach Anspruch 1 von Verbindungen der Formel I,
worin
Het für eine Gruppe der allgemeinen Formel: 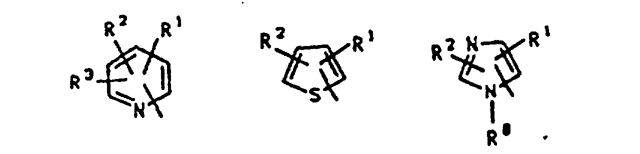 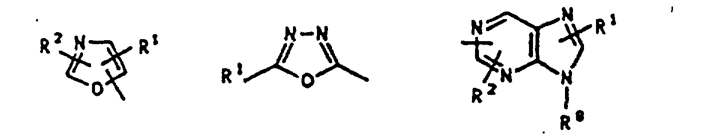 steht.

3. Verwendung nach Anspruch 2 von Verbindungen der Formel I,
worin
Het für einen Rest der allgemeinen Formel: 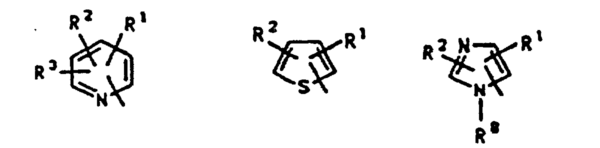 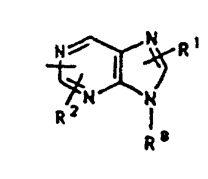 steht.

4. Verwendung nach Anspruch 2 von Verbindungen der Formel I, worin
Het für einen Rest der allgemeinen Formel: 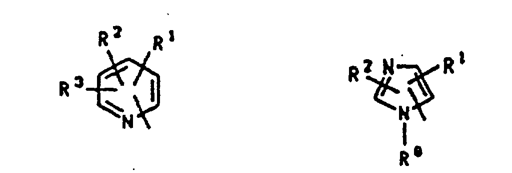 steht.

5. Verwendung nach einem der vorhergehenden Ansprüche von Verbindungen der Formel I,
worin
A für eine C₁-C₈-Alkylengruppe steht, die gegebenenfalls O, S oder CONR⁴ umfaßt, und
Ar für Phenyl oder Pyridyl steht, das gegebenenfalls einen oder zwei Substituenten aufweist, die unabhängig voneinander ausgewählt sind unter H, C₁-C₈-Alkyl, OC₁-C₈-Alkyl, CHF₂, CF₃, CN, Halogen, SO₂OR⁴ und CO₂R⁴, wobei R⁴ die in Anspruch 1 angegebenen Bedeutungen besitzt.

6. Verwendung nach Anspruch 5 von Verbindungen der Formel I, worin
A für eine C₃-C₆-Alkylengruppe steht, die gegebenenfalls S, O oder CONR⁴ enthalten kann,
Ar gegebenenfalls einen oder zwei Substituenten aufweist, die unabhängig voneinander ausgewählt sind unter H, CF₃, Halogen, C₁-C₈-Alkyl, OC₁-C₈-Alkyl und CN, und
B für 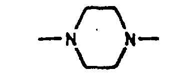 steht.

7. Verwendung nach Anspruch 6 von Verbindungen der Formel I, worin
R¹, R² und R³ jeweils unabhängig voneinander für H, NR⁴R⁵, OR⁵, C₁-C₈-Alkyl, CO₂R⁴, CF₃ oder Halogen stehen,
R⁴ für H, C₁-C₈-Alkyl steht,
R⁵ für H, C₁-C₈-Alkyl oder CO-C₁-C₈-Alkyl steht, und
R⁸ für H oder C₁-C₈-Alkyl steht.

8. Verwendung nach einem der vorhergehenden Ansprüche von Verbindungen der Formel I,
worin
A für eine C₃-C₆-Alkylengruppe steht, die gegebenenfalls S, O oder CONH enthalten kann,
B für 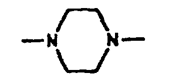 steht und
der oder die Substituenten des Restes Ar unabhängig voneinander ausgewählt sind unter H, CHF₂, C₁-C₄-Alkyl oder CF₃.

9. Verwendung nach einem der vorhergehenden Ansprüche von Verbindungen der Förmel Ia: 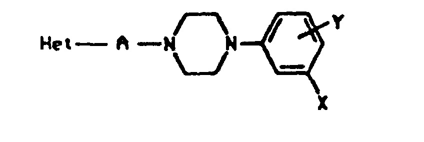 worin
Het und A die in einem der vorhergehenden Ansprüche angegebenen Bedeutungen besitzen und X und Y wie die Substituenten des Restes Ar definiert sind.

10. Verwendung nach Anspruch 9 von Verbindungen der Formel Ib: 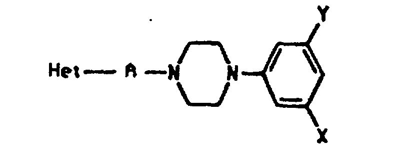 worin
Het, A, X und Y die in Anspruch 9 angegebenen Bedeutungen besitzen.

11. Verwendung nach Anspruch 9 oder 10, wobei Y für H oder C₁-C₄-Alkyl und X für CF₃, CHF₂, CN, C₁-C₄-Alkyl steht.

## Claims

1. The use of compounds of the formula I:
Het-A-B-Ar
where
A is a straight-chain or branched C₁-C₁₈-alkylene group which may comprise at least one group which is selected from among O, S, NR⁴, CONR⁴, NR⁴CO, COO, OCO and a double or triple bond,
B is a radical of the formula: 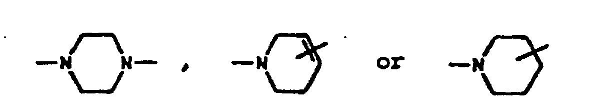
Ar is phenyl, pyridyl, pyrimidyl or triazinyl, where Ar may have one to four substituents which are, independently of one another, selected from among OR⁴, C₁-C₈-alkyl, C₂-C₈-alkenyl, C₂-C₈-alkynyl, halogen, CN, CO₂R⁴, NO₂, SO₂R⁴, SO₃R⁴, NR⁴R⁵, SO₂NR⁴R⁵, SR⁴, CF₃, CHF₂, a 5- or 6-membered carbocyclic, aromatic or non-aromatic ring and a 5- or 6-membered heterocyclic, aromatic or non-aromatic ring having 1 to 3 hetero atoms which are selected from among O, S and N, where the carbocyclic or the heterocyclic ring is unsubstituted or substituted by C₁-C₈-alkyl, halogen, OC₁-C₈-alkyl, OH, NO₂ or CF₃, and where Ar may also be fused to a carbocyclic or heterocyclic ring of the type defined above,
Het is a group which is selected from among 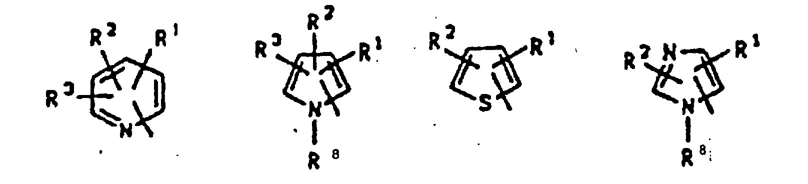 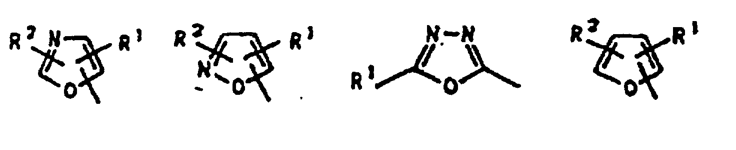 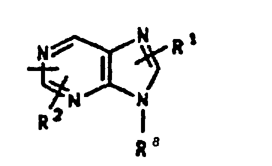 where
R¹, R² and R³ are, independently of one another, H, halogen, OR⁵, NR⁴R⁵, SR⁴, CF₃, CN, CO₂R⁴ or C₁-C₈-alkyl which is unsubstituted or substituted by OH, OC₁-C₈-alkyl or halogen,
R⁴ is H or C₁-C₈-alkyl which is unsubstituted or substituted by OH, OC₁-C₈-alkyl or halogen;
R⁵ has the meanings indicated for R⁴ or is COR⁴ or CO₂R⁴;
R⁸ has the meanings indicated for R⁵,
and the salts thereof with physiologically tolerated acids,
for the production of a pharmaceutical composition for treating schizophrenia, depression, neuroses and psychoses.

2. The use as claimed in claim 1 of compounds of the formula I
where
Het is a group of the general formula:

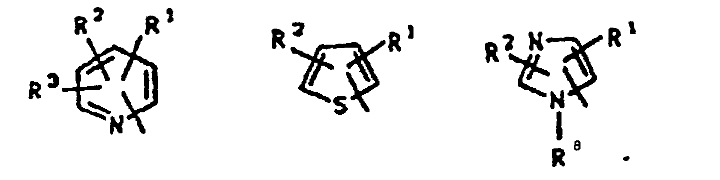

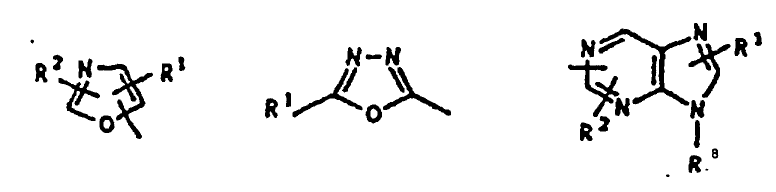

3. The use as claimed in claim 2 of compounds of the formula I
where
Het is a radical of the general formula:

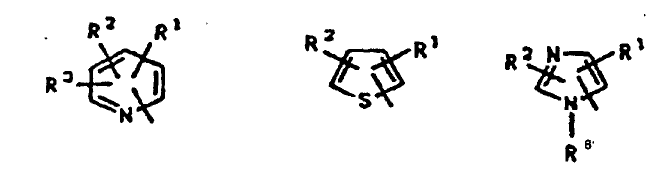

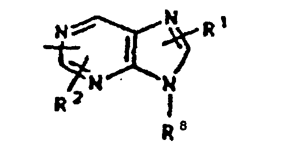

4. The use as claimed in claim 2 of compounds of the formula I
where
Het is a radical of the general formula:

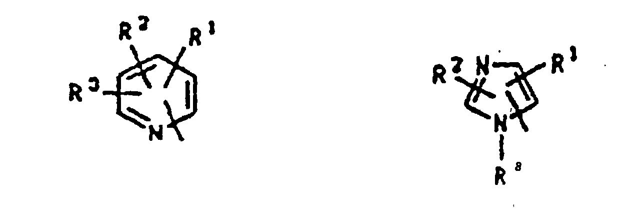

5. The use as claimed in any of the preceding claims of compounds of the formula I
where
A is a C₁-C₈-alkylene group which may comprise O, S or CONR⁴, and
Ar is phenyl or pyridyl which may have one or two substituents which are, independently of one another, selected from among H, C₁-C₈-alkyl, OC₁-C₈-alkyl, CHF₂, CF₃, CN, halogen, SO₂OR⁴ and CO₂R⁴, where R⁴ has the meanings indicated in claim 1.

6. The use as claimed in claim 5 of compounds of the formula I
where
A is a C₃-C₆-alkylene group which may contain S, O or CONR⁴,
Ar may have one or two substituents which are, independently of one another, selected from among H, CF₃, halogen, C₁-C₈-alkyl, OC₁-C₈-alkyl and CN, and
B is

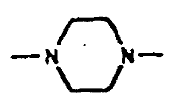

7. The use as claimed in claim 6 of compounds of the formula I
where
R¹, R² and R³ are each, independently of one another, H, NR⁴R⁵, OR⁵, C₁-C₈-alkyl, CO₂R⁴, CF₃ or halogen,
R⁴ is H or C₁-C₈-alkyl,
R⁵ is H, C₁-C₈-alkyl or CO-C₁-C₈-alkyl, and
R⁸ is H or C₁-C₈-alkyl.

8. The use as claimed in any of the preceding claims of compounds of the formula I
where
A is a C₃-C₆-alkylene group which may contain S, O or CONH,
B is 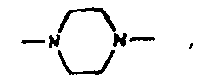 and
the substituent or substituents of the radical Ar are, independently of one another, selected from among H, CHF₂, C₁-C₄-alkyl or CF₃.

9. The use as claimed in any of the preceding claims of compounds of the formula Ia: 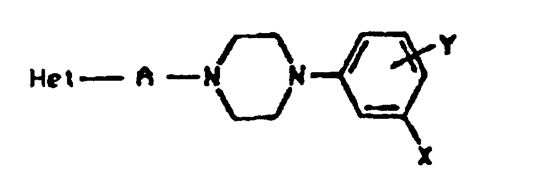 where
Het and A have the meanings stated in any of the preceding claims, and X and Y are as defined for the substituents of the radical Ar.

10. The use as claimed in claim 9 of compounds of the formula Ib: 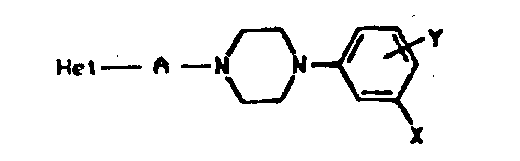 where
Het, A, X and Y have the meanings stated in claim 9.

11. The use as claimed in claim 9 or 10, where Y is H or C₁-C₄-alkyl and X is CF₃, CHF₂, CN, C₁-C₄-alkyl.

## Revendications

1. Utilisation de composés de formule I:
Het-A-B-Ar
où
A désigne un groupe alkylène en C₁-C₁₈ en chaîne droite ou ramifiée, qui peut éventuellement comporter au moins un groupe qui est choisi parmi O, S, NR⁴, CONR⁴, NR⁴CO, COO, OCO et une liaison double ou triple,
B désigne un reste de formule: 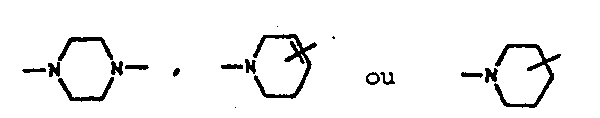
Ar représente un phényle, pyridyle, pyrimidyle ou triazinyle, tandis que Ar peut éventuellement présenter un à quatre substituants, qui sont choisis indépendamment l'un de l'autre parmi OR⁴, alkyle en C₁-C₈, alcényle en C₂-C₈, alcynyle en C₂-C₈, halogéno, CN, CO₂R⁴, NO₂, SO₂R⁴, SO₃R⁴, NR⁴R⁵, SO₂NR⁴R⁵, SR⁴, CF₃, CHF₂, un cycle carbocyclique à 5 ou 6 chaînons, aromatique ou non-aromatique, et un cycle hétérocyclique à 5 ou 6 chaînons, aromatique ou non-aromatique, ayant 1 à 3 hétéroatomes, qui sont choisis parmi O, S et N, tandis que le cycle carbocyclique ou le cycle hétérocyclique est éventuellement substitué par des groupes alkyle en C₁-C₈, halogéno, O-alkyle en C₁-C₈, OH, NO₂ ou CF₃, et tandis que Ar peut éventuellement encore être condensé avec un cycle carbocyclique ou hétérocyclique du type défini plus haut,
Het désigne un groupe qui est choisi parmi 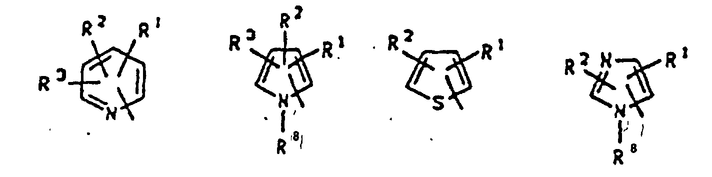 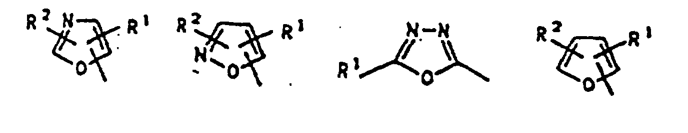 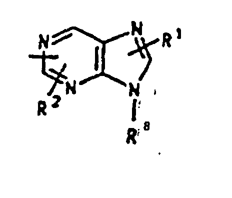
où
R¹, R² et R³ représentent indépendamment l'un de l'autre H, halogéno, OR⁵, NR⁴R⁵, SR⁴, CF₃, CN, CO₂R⁴ ou alkyle en C₁-C₈, qui est éventuellement substitué par OH, O-alkyle en C₁-C₈ ou halogéno,
R⁴ désigne H ou alkyle en C₁-C₈, qui est éventuellement substitué par OH, O-alkyle en C₁-C₈ ou halogéno;
R⁵ possède les significations indiquées pour R⁴ ou désigne COR⁴ ou CO₂R⁴;
R⁸ possède les significations indiquées pour R⁵,
et leurs sels avec des acides physiologiquement acceptables,
pour la préparation d'un produit pharmaceutique pour le traitement de schizophrénie, de dépressions, de névroses et de psychoses.

2. Utilisation selon la revendication 1 de composés de formule I où
Het désigne un groupe de formule générale:

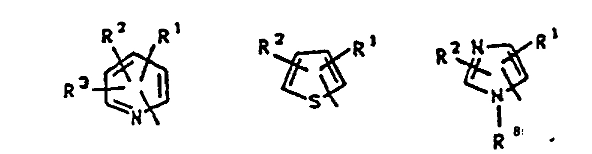

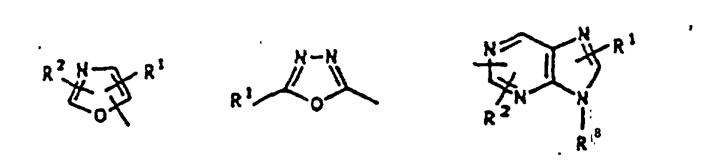

3. Utilisation selon la revendication 2 de composés de formule I, dans laquelle
Het désigne un reste de formule générale:

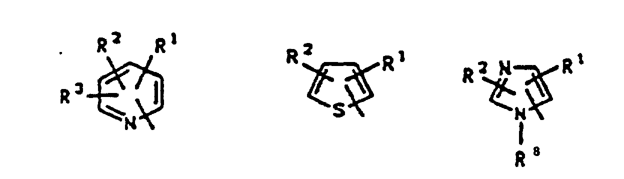

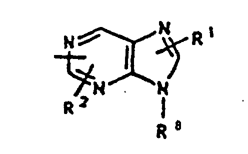

4. Utilisation selon la revendication 2 de composés de formule I, dans laquelle
Het désigne un reste de formule générale:

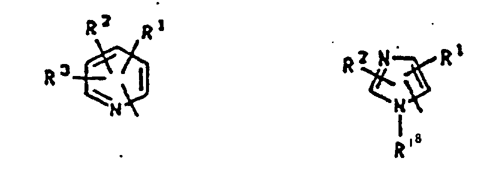

5. Utilisation selon l'une des revendications précédentes de composés de formule I, dans laquelle
A désigne un groupe alkylène en C₁-C₈, qui comporte éventuellement O, S ou CONR⁴, et
Ar représente un phényle ou un pyridyle, qui présente éventuellement un ou deux substituants qui sont choisis indépendamment l'un de l'autre parmi H, alkyle en C₁-C₈, O-alkyle en C₁-C₈, CHF₂, CF₃, CN, halogéno, SO₂OR⁴ et CO₂R⁴, tandis que R⁴ possède les significations indiquées dans la revendication 1.

6. Utilisation selon la revendication 5 de composés de formule I, dans laquelle
A désigne un groupe alkylène en C₃-C₆, qui peut éventuellement contenir S, O ou CONR⁴, et
Ar présente éventuellement un ou deux substituants qui sont choisis indépendamment l'un de l'autre parmi H, CF₃, halogéno, alkyle en C₁-C₈, O-alkyle en C₁-C₈ et CN, et
B désigne

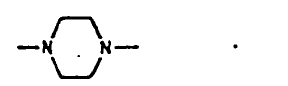

7. Utilisation selon la revendication 6 de composés de formule I, dans laquelle
R¹, R² et R³ représentent chacun, indépendamment l'un de l'autre, H, NR⁴R⁵, OR⁵, alkyle en C₁-C₈, CO₂R⁴, CF3 ou halogéno,
R⁴ désigne H, alkyle en C₁-C₈,
R⁵ représente H, alkyle en C₁-C₈ ou CO-alkyle en C₁-C₈, et
R⁸ désigne H ou alkyle en C₁-C₈.

8. Utilisation selon l'une des revendications précédentes de composés de formule I, dans laquelle
A désigne un groupe alkylène en C₃-C₆, qui peut éventuellement contenir S, O ou CONH,
B représente 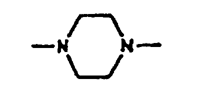 et
le ou les substituants du reste Ar sont choisis indépendamment l'un de l'autre parmi H, CHF₂, alkyle en C₁-C₄ ou CF₃.

9. Utilisation selon l'une des revendications précédentes de composés de formule Ia: 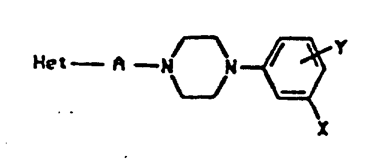 où
Het et A possèdent les significations indiquées dans les revendications précédentes, et X et Y sont définis comme les substituants du reste Ar.

10. Utilisation selon la revendication 9 de composés de formule Ib: 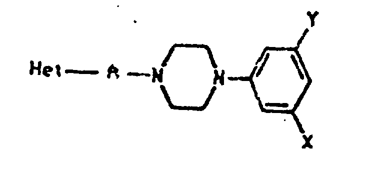 où
Het, A, X et Y possèdent les significations indiquées dans la revendication 9.

11. Utilisation selon la revendication 9 ou 10, dans laquelle Y désigne H ou alkyle en C₁-C₄, et X représente CF3, CHF2, CN, alkyle en C₁-C₄.
